# EUROPEAN PATENT APPLICATION

(11) **EP 1 837 327 A1**
(43) Date of publication of application: **26.09.2007**
(21) Application number: 06005917.7
(22) Date of filing: 23.03.2006
(51) Int. Cl.: C07C 67/52, C07C 67/31, C07C 69/738

(54) **Method for preparing pure fenofibrate**

(71) Applicant: SOLMAG S.p.A., 26837 Mulazzano LO (IT)
(72) Inventor: Tubertini, Paolo, Cassino d'Alberi 26837 Mulazzano (LO) (IT); Vecchio, Emilio, Cassino d'Alberi 26837 Mulazzano (LO) (IT)
(74) Representative: Minoja, Fabrizio

(57) **Abstract**

A process for the preparation of fenofibrate is disclosed. The process, consisting in crystallisation in specific conditions from C₁-C₄ alcohols or ketones, allows to obtain fenofibrate having a polymeric impurity content lower than 0.5%.

## Description

### Field of the Invention

The present invention provides a method for the preparation of pure Fenofibrate, and a method of purification thereof.

### Background of the invention

2-[4-(4-chlorobenzoyl)phenoxy]-2-methyl propanoic acid 1-methyl ethyl ester, also known as Fenofibrate, having formula (I) 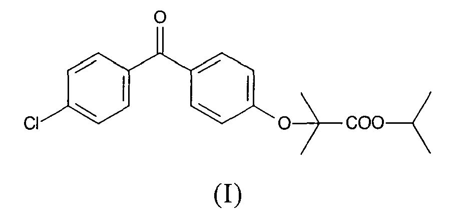 was disclosed in US 4,058,552 as a cholesterol lowering and antihyperlipidemic drug.

Several processes for the preparation of Fenofibrate are known for instance from US 4,739,101, EP 2151, and US 6,897,333. Fenofibrate is prepared by reacting a compound of formula (II) 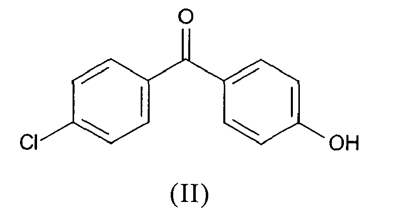 with a bromo derivative of formula (III), 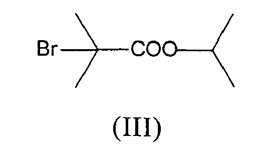 in the presence of bases such as K₂CO₃ or KHCO₃, in presence or absence of solvents, to give a compound of formula (I). Typically, in the above mentioned patents, the reaction is carried our at temperatures ranging from 80°C to 160°C and for a time ranging from few hours to a few days. The final compound is then obtained by crystallization from aqueous isopropanol.

However, during the reaction between a compound of formula (II) and the compound of formula (III) considerable amounts (up to 1%) of an impurity of polymeric nature, namely a poly(isopropyl methacrylate), of formula (IV) (n is a number giving an average molecular weight ranging from 50.000 to 200.000) 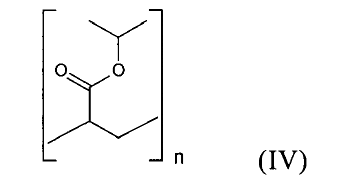 are formed. Said impurity is difficult to detect, and it is hardly removed from the final compound during the final crystallization, if performed as described in the known methods.

There is therefore the need for an improved process, able to minimize the formation of impurities during the reaction, or to remove the impurity during the final crystallization and/or purification.

### Summary of the invention

The present invention concerns an improved process for the preparation of Fenofibrate, allowing the reduction of the impurity content to less than 0.5%, preferably less than 0.1%.

The process of the invention comprises
i) Reacting a compound of formula (II), 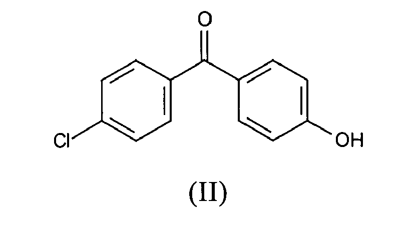 with a compound of formula (III) 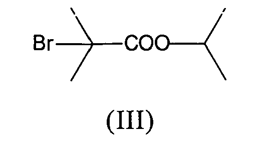
ii) Cooling the reaction mixture at a temperature ranging from 40°C to 80°C;
iii) Adding solvents selected from C₁-C₄ alcohols and ketones;
iv) Crystallisation of fenofibrate by cooling first to a temperature comprised between 10 and 50°C, stirring the mixture, filtering off the salts, continuing crystallisation overnight at room temperature at 0-5°C;
v) Recovering the fenofibrate crystals by filtration, washing and drying at a temperature of about 60°C.

A further object of the invention is fenofibrate obtainable buy said process, characterised by a content of polymeric impurity of formula IV lower than 0.5% 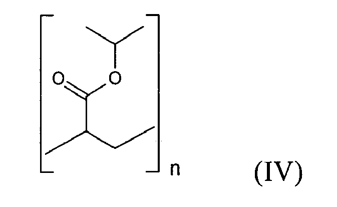 wherein n has the same meaning defined above.

### Detailed description of the invention

The reaction of compound II with compound III is carried out according to known methods, for instance as disclosed in US 6,897,333, in solvents (e.g. alcohols such isopropanol) and in the presence of alkali metal bicarbonates or other bases.

The reaction mixture is then cooled to at a temperature ranging from 40°C to 80°C, preferably from 50°C to 70°C, and most preferably at about 60°C.

Isopropanol and acetone are preferred solvents for the subsequent step.

A decolorizing agent such as charcoal may be optionally added before the further cooling at a temperature ranging from 10 and 50°C, preferably at about 40°C. After stirring the mixture for some time, preferably from few minutes to two hours, and most preferably for about 30', the salts and the decolorizing agent are filtered off, optionally in the presence of a filter aid, washing the solid on the filter with the same solvents used in the previous step. Fenofibrate is then crystallized by cooling the filtrate at room temperature overnight, and at 0-5°C for a few hours.

Fenofibrate crystals are filtered, washed, and then dried at a temperature of about 60°C for a few hours, to eventually obtain pure fenofibrate with very low impurity content.

Alternatively, crude fenofibrate, containing higher amounts of said polymeric impurity, may be further purified by:
i) Suspending fenofibrate in ketones, preferably C₃-C₆ ketones,
ii) Heating the suspension to reflux until complete dissolution,
iii) Adding a C₁-C₄ alcohol,
iv) Cooling the solution to a temperature from 0 to 40°C, preferably from 10 to 30°C, and stirring the suspension overnight at the same temperature, and
v) Further cooling the mixture to a temperature from 0°C to 5°C, and then filtering the obtained crystals, collecting them, and eventually drying the resulting pure fenofibrate at about 60°C overnight.

The following examples further illustrate the process of the invention.

### Example 1

200 g of 4-chloro-4-hydroxybenzofenone, potassium bicarbonate (156 g), 360 g of isopropyl α-bromo isobutyrate and isopropanol (400 ml) were charged in a round bottom flask. The mixture was heated to reflux temperature for 48 hours, with stirring. After reaction completion, the reaction mixture was cooled at about 60°C, and isopropanol (560 ml), acetone (240 ml), and a decolorizing agent (4.5 g of activated carbon) were added. The suspension was further cooled to about 40°C, and stirring was continued for about 30 minutes. The suspension was filtered, and the solid was washed on the filter with a mixture of isopropanol and acetone. The filtrate was let to stand overnight at room temperature, and then stirred for additional 3-4 hours at 0-5°C. Precipitated pure fenofibrate was filtered, washed on the filter with isopropanol and water. The compound was collected from the filter and dried at about 60° for 12 hours, to yield 220 g of pure fenofibrate.

### Example 2

Fenofibrate (100 g) was suspended in acetone (100 ml). The suspension was refluxed till complete solution. Isopropanol (300 ml) was then added in about 30 minutes, at about 40-45°C. The mixture was cooled to 15-20°C and stirring overnight. The suspension was then stirred at 0-5°C for about 4-6 hours, filtered and washed with isopropanol. The product was collected from the filter, and dried at about 60°C, to yield about 80 g of pure fenofibrate.

## Claims

1. Fenofibrate **characterised by** a content of polymeric impurity of formula IV lower than 0.5% 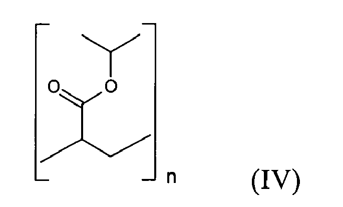 wherein n is a number giving an average molecular weight ranging from 50.000 to 200.000.

2. A process for the preparation of fenofibrate which comprises
i) Reacting a compound of formula (II), 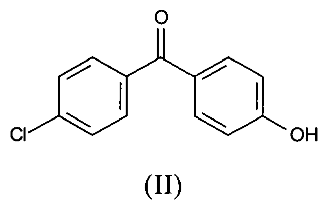 with a compound of formula (III) 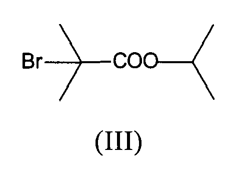
ii) Cooling the reaction mixture at a temperature ranging from 40°C to 80°C;
iii) Adding solvents selected from C₁-C₄ alcohols and ketones;
iv) Crystallisation of fenofibrate by cooling first to a temperature comprised between 10 and 50°C, stirring the mixture, filtering off the salts, continuing crystallisation overnight at room temperature at 0-5°C;
v) Recovering the fenofibrate crystals by filtration, washing and drying at a temperature of about 60°C.

3. A process according to claim 2 wherein the reaction mixture is cooled to a temperature from 50°C to 70°C.

4. A process according to claim 3 wherein the reaction mixture is cooled to 60°C.

5. A process according to claim 2-4 wherein the solvents in step iii) are selected from acetone and isopropanol.
